# EUROPEAN PATENT SPECIFICATION

(11) **EP 2 178 510 B1**
(45) Date of publication and mention of the grant of the patent: **09.04.2014**
(21) Application number: 08775226.7
(22) Date of filing: 18.07.2008
(51) Int. Cl.: A61K 9/20, A61K 9/48, A61K 31/4184

(54) **STABLE SOLID PHARMACEUTICAL COMPOSITION COMPRISING CANDESARTAN OR PHARMACEUTICALLY ACCEPTABLE FORMS THEREOF**
STABILE FESTE PHARMAZEUTISCHE ZUSAMMENSETZUNG MIT CANDESARTAN ODER PHARMAZEUTISCH ANNEHMBARE FORMEN DAVON
COMPOSITION PHARMACEUTIQUE SOLIDE STABLE COMPRENANT DU CANDESARTAN OU SES FORMES PHARMACEUTIQUEMENT ACCEPTABLES

(30) Priority: 20.07.2007 SI 200700187; 18.10.2007 EP 07118772
(43) Date of publication of application: 28.04.2010
(73) Proprietor: KRKA, D.D., Novo Mesto, 8501 Novo mesto (SI)
(72) Inventor: SEDMAK, Gregor, 1000 Ljubljana (SI); TURK, Urska, 8000 Novo Mesto (SI); SMRKOLJ, Matej, 1420 Trbovlje (SI); ZUPANCIC, Silvo, 8000 Novo Mesto (SI)
(74) Representative: HOFFMANN EITLE
(86) International application number: PCT/EP2008/059451
(87) International publication number: WO 2009/013237

(56) References cited:
- WO-A-98/53798
- WO-A-2005/084648
- WO-A-2005/117591
- WO-A-2007/093305
- WO-A-2007/147514
- WO-A-2008/065097
- DATABASE CA [Online] CHEMICAL ABSTRACTS SERVICE, COLUMBUS, OHIO, US; 20 April 2007 (2007-04-20), GUO, JIANHUI ET AL: "Controlled-release drug composition containing angiotensin II1 receptor antagonist" XP002478507 retrieved from STN Database accession no. 2007:439088 & CN 1 947 708 A (SHANGHAI ALLIST PHARMACEUTICALS, INC., PEOP. REP. CHINA) 18 April 2007 (2007-04-18)
- HIROKAZU MATSUNAGA ET AL: "Solid-state characterization of candesartan cilexetil (TCV-116): crystal structure and molecular mobility" 1 February 1999 (1999-02-01), CHEMICAL AND PHARMACEUTICAL BULLETIN, PHARMACEUTICAL SOCIETY OF JAPAN, TOKYO, JP, PAGE(S) 182 - 186 , XP002957606 ISSN: 0009-2363 cited in the application the whole document

## Description

### Field of the invention

The present invention belongs to the field of pharmaceutical industry, particularly to the field of solid pharmaceutical compositions. Specifically, the invention relates to a stable solid pharmaceutical composition comprising candesartan or its pharmaceutically acceptable forms and at least one plasticizer.

### Background of the invention

Candesartan is a selective antagonist of subtype 1 of the angiotensin II receptor (AT₁-antagonist). It falls in the class of drugs of benzimidazole-7-carboxylic acids and derivatives thereof. These agents exhibit a strong and more effective hypotensive action and are less likely to cause coughing as a side effect when compared to the class of ACE inhibitors.

In general, the formulation of candesartan or its pharmaceutically acceptable forms for effective oral administration to a subject has hithereto been complicated by the unique physical and chemical properties of the compound. It is known that candesartan or its pharmaceutically acceptable forms are stable against temperature, moisture and light when they are stored individually in the solid state while when they are incorporated into a pharmaceutical composition the decomposition of the active ingredient is observed. Up to now this problem was solved by several different ways with the main stress on solving the stability problems.

A number of different types of materials and approaches has already been tried in order to find the solution for stabilizing candesartan cilexetil in a solid pharmaceutical composition.

EP 546358 B1 solves the problem of decomposition of candesartan and/or its pharmaceutically acceptable salts by incorporating an oily substance having a low melting point into the formulation.

WO 2005/070398 discloses the dispersing of candesartan cilexetil in a solution comprising a co-solvent and water to form a suspension.

A pharmaceutical formulation containing candesartan cilexetil and one or more fatty acids is disclosed in WO 2005/079751.

As disclosed in WO 2005/072709 and WO 2006074218 the problem could be solved by forming nanoparticles or microparticles of the drug.

WO 2005/084648 discloses the use of water-soluble polymers to form a stable pharmaceutical composition of candesartan cilexetil with respect to the levels of impurities.

WO 2006/079496 discloses as a stabilizing agent a member of a group of materials known as natural gums, for example agar, xanthan gum, gum arabic (acacia), ceratonia, carrageenan.

WO 2006/079496 further discloses that candesartan cilexetil can be de-stabilized during compression molding of tablets and for that only gentle manipulation of that step has to be undertaken. Such a gentle manipulation was defined as that the main pressure during compression molding is at maximum 20 kN, preferably 10 kN.

US 2007/0014864 A, US 2007/0014853 A and US 2007/0014854 A disclose pharmaceutical granules containing an active substance having poor water solubility and at least one pharmaceutically acceptable sugar.

In particular, a constant need exists for effective, orally deliverable solid pharmaceutical composition comprising candesartan or its pharmaceutically acceptable forms possessing one or more of the following characteristics:
- appropriate disintegration time of the pharmaceutical composition,
- appropriate dissolution profile of the active ingredient,
- appropriate processability,
- ordinary used and cost effective pharmaceutically acceptable excipients,
- appropriate economics of the technological process for the preparation of the final drug product,
- appropriate chemical and physical stability of the final drug product,
- appropriate bioavailability of the final drug product,

and being prepared by the technological process of wet and dry granulation in order to:
- increase the uniformity of active ingredient distribution in the formulation,
- densify the granulate, as for example a mixture of an active ingredient and at least one pharmaceutically acceptable excipient,
- enhance the flow rates and rate uniformity, i.e. to improve physical characteristics,
- reduce dust,
- improve the appearance of the solid dosage form,
and wherein the active substance possesses the following characteristics:
- appropriate particle size distribution,
- appropriate solubility.

Therefore, an object underlying the present invention is to provide a stable solid pharmaceutical composition comprising candesartan or its pharmaceutically acceptable forms wherein the term stable composition refers to the total content of impurities originating from the decomposition of candesartan in the pharmaceutical composition and to the retained stable polymorphic form I of candesartan cilexetil.

### Summary of the invention

In a first aspect, the present invention relates to a stable solid pharmaceutical composition in the core, wherein the plasticizer is comprising candesartan and at least one plasticizer according to claim 1, selected from the group consisting of phthalate esters, sebacates, polyacrylate polymers, polymers and copolymers of ethylene, and/or vinyl acetate, triacetin, menthol and any mixture thereof.

Also disclosed is a stable solid pharmaceutical composition comprising candesartan or its pharmaceutically acceptable forms and at least one plasticizer selected from the group consisting of phthalate esters, sebacates, alginates, citrate esters, polyacrylate and polymethacrylate polymers, polymers and copolymers of ethylene, vinyl and/or acetate, various sugar alcohols, triacetin, menthol and any mixture thereof, wherein the total content of impurities does not exceed 10 % area, preferably 6 % area, more preferably 4 % area and most preferably 2 % area determined by liquid chromatography if such a composition is stored for 7 days at 60 °C in a closed vessel. The plasticizer is preferably selected from the group of citrate esters.

Also disclosed is the use of a plasticizer selected from the group consisting of citrate esters in a stable solid pharmaceutical composition containing candesartan cilexetil as an active ingredient to retain its stable polymorphic form I. The plasticizer is preferably triethyl citrate. ,

In a further aspect, the present invention relates to a process according to claim 12, for the preparation of a stable solid pharmaceutical composition comprising candesartan and at least one plasticizer as described above by a process comprising a step selected from the group consisting of wet granulation, dry granulation and direct compression.

In yet another aspect, the present invention relates to a method of treating hypertension, cardiac diseases, cerebral apoplexy or renal diseases in a patient in need thereof by administering a stable solid pharmaceutical composition comprising candesartan according to the present invention.

### Brief description of drawings

Figure 1: Particle size distribution of milled candesartan cilexetil material
Figure 2: Particle size distribution of unmilled candesartan cilexetil material
Figure 3: Microscopic picture of milled candesartan cilexetil material
Figure 4: Microscopic picture of unmilled candesartan cilexetil material
Figure 5: Comparison: Powder diffraction patterns of stable solid pharmaceutical compositions comprising candesartan cilexetil, and of candesartan cilexetil as pure substance, respectively.

### Detailed description of the invention

Therefore, an object underlying the present invention is to provide a stable solid pharmaceutical composition comprising candesartan or its pharmaceutically acceptable forms. In contrast to the pharmaceutical compositions according to the prior art described above, the present invention relates to a stable solid pharmaceutical composition wherein the stability of candesartan or its pharmaceutically acceptable forms is achieved in a very simple way, that is by the addition of an appropriate plasticizer. The pharmaceutical composition according to the present invention offers the advantage of a low content of total impurities originating from the decomposition of candesartan and of retaining the stable polymorphic form I of candesartan cilexetil.

A person skilled in the art is aware of a number of substances defined as stabilizing agents in the pharmaceutical field, as for example natural gums, that could be used in designing a stable solid pharmaceutical composition comprising candesartan or its pharmaceutically acceptable forms. We found that said substances do not provide adequate stabilization of candesartan cilexetil in a solid pharmaceutical composition.

Further, during our extensive experimental work we found that any kind of rough physical manipulation of candesartan or its pharmaceutically acceptable forms can substantially reduce its stability. We found that even the method of encapsulation can destabilize the active substance in the solid pharmaceutical composition. In order to lower the frictional forces between individual particles present in the solid composition of candesartan or its pharmaceutically acceptable forms, a person skilled in the art can try substances already known in the state of the art as tablet or capsule lubricant. Examples of said substances are calcium and magnesium stearate, sodium stearyl fumarate, stearic acid and the like. Again, we found that said substances do not provide sufficient stabilization of candesartan or its pharmaceutically acceptable forms in a solid pharmaceutical composition as well.

Further, substances known in the art as surfactants and emulsifying agents, as for example docusate sodium, calcium steroyl-2-lactylate, benzalkonium chloride, cetrimide and sodium lauryl sulphate, and emulsifying, gelling and film-forming agents as for example hypromellose, hydroxypropyl cellulose, gelatin, pectin, alginates like sodium and potassium alginate and hypromellose acetate succinate were tested wether they lower the frictional forces between individual particles in a stable solid composition of candesartan or its pharmaceutically acceptable forms. Again we found that none of the selected substances does provide sufficient stabilization of candesartan and its pharmaceutically acceptable forms in a solid pharmaceutical composition as well.

Surprisingly, it has been found that some of the substances, known in the pharmaceutical field as plasticizers, do provide sufficient stabilization of candesartan and its pharmaceutically acceptable forms in a solid pharmaceutical composition.

The general definition used to describe plasticizers refers to pharmaceutically acceptable excipients that increase the plasticity or the ductility of the materials they are added to and that are generally applied in the preparation of film coatings for pharmaceutical dosage forms.

The term plasticizer used herein, however, refers to a pharmaceutically acceptable excipient that is added to the core of a pharmaceutical composition and not into the coating thereof. Such a plasticizer is used to increase the plasticity and/or decrease the friction between the individual particles (constituents) of a pharmaceutical formulation on the microscopic level due to its lubrication properties.

Thus, in one aspect, the present invention relates to a stable solid pharmaceutical composition comprising candesartan and at least one plasticizer in the core, wherein the plasticizer is selected from the group consisting of phthalate esters, sebacates, polyacrylate polymers, polymers and copolymers of ethylene, and/or vinyl acetate, triacetin, menthol and any mixture thereof.

The plasticizer used in the stable solid pharmaceutical composition according to the present invention may be selected from, but not limited to:
- Phthalate esters (such as for example phthalate esters like polyvinyl acetate phthalate, cellulose acetate phthalate, dibutyl phthalate, diethyl phthalate, dimethyl phthalate, hypromellose phthalate),
- Sebacates (such as for example dibutyl sebacate and diethyl sebacate),
- Polyacrylate polymers,
- Polymers and copolymers of ethylene, and/or vinyl acetate (such as for example polyethylene, ethylene vinyl acetate),
- triacetin, menthol.

Preferably, the plasticizer is selected from the group consisting of phthalate esters, sebacates, polyacrylate polymers, triacetin, menthol and any mixture thereof. More preferably, the plasticizer is selected from the group consisting of menthol and triacetin and any mixture thereof.

One or more plasticizers used as a stabilizing agent in a solid pharmaceutical composition according to the present invention is present in an amount of about less than 20% [w/w] of the final pharmaceutical composition and preferably in an amount of about between 0.5 and 10% [w/w]of the final pharmaceutical composition.

The term candesartan or its pharmaceutically acceptable forms used herein refers to a prodrug that is hydrolyzed to candesartan during absorption from the gastrointestinal tract, more particularly it refers to candesartan cilexetil. Additionally, the term candesartan or its pharmaceutically acceptable forms used herein refers to candesartan or its pharmaceutically acceptable esters as for example cilexetil, or to its pharmaceutically acceptable salts, as for example alkaline salts or ammonium salts, preferably the active ingredient is candesartan cilexetil.

The concentration of candesartan or its pharmaceutically acceptable forms in the pharmaceutical composition may be between about 1 to about 99% [w/w], particularly between about 1 to about 30% [w/w] and more particularly between about 1 to about 20% [w/w] determined by HPLC.

The average particle size of candesartan or its pharmaceutically acceptable forms may be between 1 and 50 µm, preferably between 1 and 40 µm, more preferably less than 20 µm, determined by a laser diffraction method with a Malvern Mastersizer 2000 instrument equiped with the Hydro S dispersion unit, which is appropriate for measuring particle size distributions in the range of 20 nm to 2000 µm. Isopar L is used as dispersant. Usually, 100-800 mg of the substance is dispersed in 5-10 ml of a 2% solution of epicurone (lecithin) in isopar L to get a homogeneous sample suspension. The measurement is started after adding the sample suspension into the dispersion unit, homogenizing by sonication for an appropriate time to brake-up agglomerates and recirculating at a stirrer/pump speed rate of about 50-70% of maximum speed. The following particel size (D10, D50 and D90) values were measured for milled and unmilled candesartan cilexetil material:

**Table 1. D10, D50 and D90 values of milled and unmilled candesartan cilexetil material**

| | **Milled** | **Unmilled** |
|---|---|---|
| **Mean particle diameter** | 4µm | 57/15µm (with ultrasonication) |
| **D10** | 1.1µm | 3.1µm |
| **D50** | 2.8µm | 10.5µm |
| **D90** | 6.8µm | 30.6µm |
| **Specific surface area** | 7.2 m²/g | 4.2 m²/g |

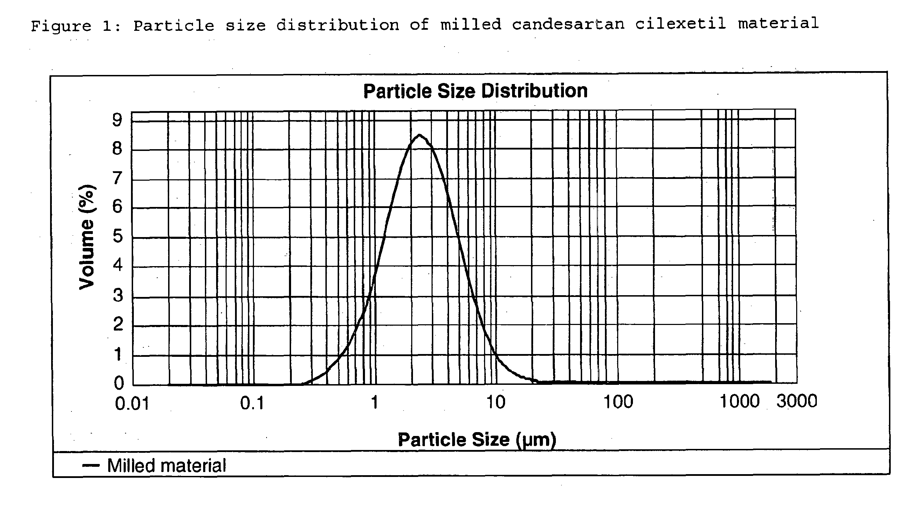

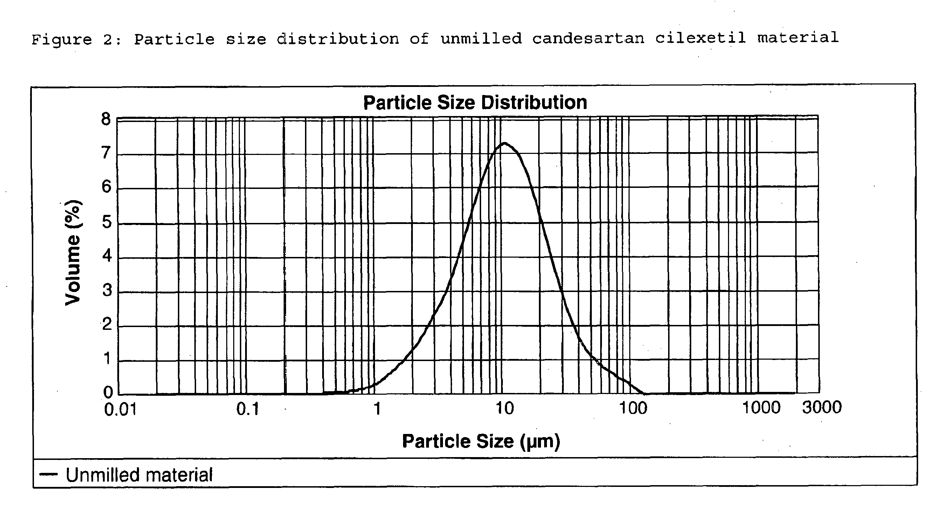

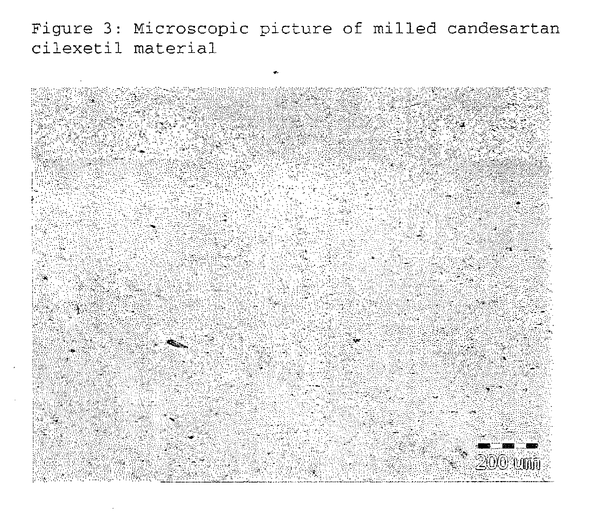

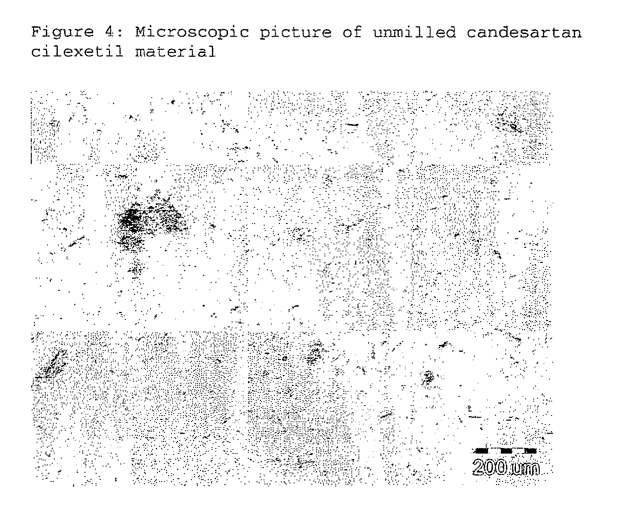

D10/50/90 means that at least 10/50/90 % by volume of the particles have a particle size below the specified value. The good homogeniety of the respective size distribution can be taken form the size distribution profiles as shown in Figures 1 and 2. In addition, the microscopic pictures of the milled and the unmilled candesartan cilexetil material (Figures 3 and 4) confirm the homogenous size distribution especially of the milled material (Figure 3) present in the more prefered size of less than 20 µm (cf. above).

The candesartan or its pharmaceutically acceptable forms present in the stable solid pharmaceutical composition according to the present invention can be prepared by any method known from the state of the art as for example disclosed in Jornal of pharmacy and pharmacology. 2005, 57, 273-285, EP 459136B1, WO 2003/014112, WO 2005/021535, WO 2005/051928, WO 2005/051929, EP 1555260 B1, WO 2006/015134, WO 2006/050922, WO 2006/063578, WO 2006/067215, WO 2006/103068, WO 2006/125592, WO 2007/074399, WO 2006/134078, WO 2007/014412, WO 2007/054965, EP 1777224, WO 2007/042161, WO 2007/048361, WO 2005/037821, WO 2006/076710, WO 2005/111021, WO 2006/122254, PCT/EP2007/057803, PCT/EP2007/057804 and can be in any known stable polymorphic forms.

Preferably, candesartan cilexetil exists in polymorphic form I or in amorphous form, more preferably in polymorphic form I as defined by Chem. Pharm. Bull. 47 (2) 182-186 (1999).

The stable solid pharmaceutical composition according to the present invention may optionally further contain one or more other active ingredients. Suitable other active ingredients can include but are not limited to other angiotensin-II-receptor-antagonists, diuretics, sympathoplegic agents, Ca-channel blockers, vasodilators, ACE inhibitor, angiotensin receptor antagonists, inhibitors of HMG-CoA reductase and any other pharmaceutically acceptable combination, particularly the other active agent is selected from the group consisting of, but is not limited to, angiotensin-II-receptor-antagonists, Ca-channel blockers, diuretics and inhibitors of HMG-CoA reductase. When two or more other active ingredients are present in the stable pharmaceutical composition according to the present invention, they can be present in the same phase (in the intragranular phase, i.e. in the granulate, or in the extragranular phase) as candesartan or its pharmaceutically acceptable forms or in a different phase as candesartan or its pharmaceutically acceptable forms. Additionally, candesartan or its pharmaceutically acceptable forms and one or more other active ingredient can be present partly in the intragranular phase and partly in the extragranular phase. The concentration of candesartan or its pharmaceutically acceptable forms and of one or more other active ingredients in the pharmaceutical composition may be between about 1 to about 99% [w/w], particularly between about 1 to about 30% [w/w] and more particularly between about 2 to about 20% [w/w] determined by HPLC.

The stable solid pharmaceutical composition according to the present invention may further include one or more pharmaceutically acceptable ingredients, i.e. excipients such as for example diluents, binders, disintegrants, surfactants and lubricants. Other and further excipients can also be included. The term »pharmaceutically acceptable excipients« denotes that the additives are used to convert pharmacologically active compounds into pharmaceutical dosage forms suitable for the administration to patients. Pharmaceutically acceptable excipients can be solid, liquid or semi-solid and are converted during the process into a state that allows the prepraration of a stable solid pharmaceutical composition comprising candesartan or pharmaceutically acceptable forms thereof. The pharmaceutically acceptable excipient may be present in the stable solid pharmaceutical composition according to the present invention in an amount of about 1 to about 99% [w/w], particularly from about 70 to about 99% [w/w], more particularly from about 90 to about 98% [w/w].

The particle size of the excipients used in the stable solid pharmaceutical composition according to the present invention is within the range of D90<750 µm, preferably D90<500, in order to ensure the homogeneity of the compression mixture as well as homogeneous granulation and compression. D90 means that at least 90% by volume of the particles have a particle size below the specified value.

The diluents used in the stable solid pharmaceutical composition according to the present invention may be selected from the group consisting of, but are not limited to, microcrystalline cellulose, powdered cellulose, lactose (anhydrous and monohydrate), compressible sugar, fructose, dextrates, sugar alcohols such as mannitol, sorbitol, maltitol, xylitol, lactitol, or other sugars such as saccharose, raffinose, trehalose, fructose or mixture thereof, siliconised microcrystalline cellulose, calcium hydrogenphosphate, calcium carbonate, calcium lactate or any mixture thereof, preferably, microcrystalline cellulose, lactose and mannitol, or any mixtures thereof, more preferably microcrystalline cellulose and lactose.

The binders used in the stable solid pharmaceutical composition according to the present invention may be selected from the group consisting of, but are not limited to, polyvinylpyrrolidone, microcrystalline cellulose, hydroxyethylcellulose, hydroxypropylcellulose, hydroxypropylmethylcellulose or other cellulose ethers, starch, pregelatinised starch, or polymethacrylate, or any mixtures thereof. It is preferable to use a binder with good water solubility. In view of the practical insolubility of starch in cold water, there is a strong preference for binders with a very good solubility in cold water. There is a variety of binders that have a very good solubility in water, such as for example povidone of different K-values and hydroxypropylcellulose, such as for example partially substituted poly (hydroxypropyl) ether of cellulose and/or low substituted poly(hydroxypropyl) ether of cellulose. Preferably, at least one binder is selected from polyvinylpyrrolidone and hydroxypropylcellulose.

The disintegrants used in the stable solid pharmaceutical composition according to the present invention may be selected from the group consisting of, but are not limited to, crospovidone, starch, pregelatinised starch, sodium strach glycollate, microcrystalline cellulose, carboxymethylcellulose sodium (CMC-Na) or calcium (CMC-Ca), cross-linked CMC-Na, polacrilin potassium, low-substituted hydroxypropylcellulose or any mixtures thereof. Prefereably, at least one disintegrant is selected from cross-linked CMC-Na, starch and low-substituted hydroxypropylcellulose.

The surfactants used in the stable solid pharmaceutical composition according to the present invention may be selected from the group consisting of, but are not limited to:
1. Anionic surfactants, where the hydrophilic group carries a negative charge, such as for example carbonyl (RCOO⁻), sulphonate (RSO₃⁻) or sulphate (ROSO₃⁻), examples include potassium laurate, CH₃ (CH₂) ₁₀COO⁻K⁺, and sodium lauryl sulphate, CH₃ (CH₂) ₁₁SO₄⁻Na⁺.
2. Cationic surfactants, where the hydrophilic group carries a positive charge (e.g., quaternary ammonium halides, R₄N⁺Cl⁻), examples include cetrimide, a mixture consisting mainly of tetradecyl (ca. 68% [w/w]), dodecyl (ca. 22% [w/w]), and hexadecyltrimethylammonium bromides (ca. 7% [w/w]), as well as benzalkonium chloride, a mixture of alkylbenzyldimethylammonium chlorides of the general formula [C₆H₅CH₂N⁺ (CH₃)₂R]Cl⁻, where R represents a mixture of the alkyls from C₈H₁₇ to C₁₈H₃₇.
3.Ampholytic surfactants (also called zwitterionic surfactants), where the molecule contains, or can potentially contain, both a negative and a positive charge, (e.g., sulfobetaines, RN⁺ (CH₃)₂CH₂CH₂SO₃⁻), examples include N-Dodecyl-N,N-Dimethylbetaine, C₁₂H₂₅N⁺ (CH₃)₂CH₂COO⁻.
4. Nonionic surfactants, where the hydrophilic molecule carries no charge but derives its water solubility from highly polar groups such as hydroxyl or polyoxyethylene (-OCH₂CH₂O-) groups, examples include polyoxyethylated glycol monoethers (e.g. cetomacrogol), sorbitan esters (Spans^{®}) and polysorbates (Tweens^{®}) as well as polyoxyethylene-polyoxypropylene copolymers.

The lubricants used in the stable solid pharmaceutical composition according to the present invention may be selected from the group consisting of, but are not limited to, stearic acid or stearic acid salts (such as for example magnesium stearate), magnesium palmitate, magnesium oleate, hydrogenated vegetable oil, hydrogenated castor oil, talc, sodium stearyl fumarate, macrogols or mixtures thereof. Preferably, excipients include at least one lubricant selected from stearic acid, magnesium stearate, hydrogenated vegetable oil, hydrogenated castor oil and talc, more preferably magnesium stearate, hydrogenated castor oil and talc.

Optionally, cores/tablets can be coated with conventional materials used for film coating, i.e. as described in Pharmaceutical Coating Technology (G. Cole (ed.), 1995). Film coating formulations usually contain the following components:
- polymer(s),
- plasticizer(s),
- colourant(s)/opacifier(s),
- vehicle(s).

In the film coating suspension minor quantities of flavours, surfactants and waxes can be used.

The majority of the polymers used in film coating are either cellulose derivatives, such as the cellulose ethers, or acrylic polymers and copolymers. Occasionally encountered are high molecular weight polyethylene glycols, such as for example macrogols, polyvinylpyrrolidone, polyvinylalcohol and waxy materials. Their function usually is to prevent bad mouth feel and/or taste and in some cases degradation, e.g. oxidation of the active ingredients and/or excipients used.

Typical cellulose ethers which may be applied as coatings are hydroxyethylcellulose, hydroxypropylcellulose, hydroxypropylmethylcellulose and methylcellulose. Acrylic polymers comprise a group of synthetic polymers with diverse functionalities. Some of them can be further modified to enhance swelling and permeability by the incorporation of materials such as water soluble cellulose ethers and starches in order to ensure complete disintegration/dissolution of the film.

The commonly used plasticizers can be categorized into three groups:
- polyols (glycerol, propylene glycol, macrogols),
- organic esters (phthalate esters, dibutyl sebacetate, citrate esters, triacetin),
- oils/glycerides (castor oil, acetylated monoglycerides, fractionated coconut oil).

Colourants/opacifiers are classified into several groups:
- organic dyes and their lakes,
- inorganic colours,
- natural colours.

Vehicles are materials that perform a necessary function in that that they provide the means of conveying the coating materials to the surface of the tablet or particle. The major classes of vehicles capable of being used are water, alcohols, ketones, esters and chlorinated hydrocarbons.

Different materials from each group can also be combined in defined ratios. Ready-to-use preparations, which are available on the market, can also be used as film coating suspensions.

Film coating suspensions can be prepared by using different solvents as for example water, alcohols, ketones, esters and chlorinated hydrocarbons, preferably water.

A composition of a coating suspension (calculated on dry material) which comprises:
- 1-99%, preferably 1-95% by weight of polymer,
- 1-50%, preferably 1-40% by weight of plasticizer,
- 0.1-20%, preferably 0.1-10% by weight of colourant/opacifier,
is particularly preferred.

Ingredients described as components of the coating layer, e.g. colourants, can also be incorporated in the tablet core or divided between tablet core and film coating layer.

The stable solid pharmaceutical composition according to the present invention can be in the form of tablets, pills, powders, lozenges, sacchets, soft and hard gelatine capsules, suppositories and the like. The dosage form is preferably suitable for oral application, more preferably it is in the form of tablets or capsules.

The stable solid pharmaceutical composition according to the present invention is preferably formulated in a unit dosage form, each dosage form containing about 1 to 100 mg, more usually about 1 to about 50 mg of candesartan cilexetil, preferably from about 2 to 32 mg. When one or more other active ingredients are present in the stable solid pharmaceutical composition according to the present invention said other active ingredient can be present in an amount of 6.25 to 50 mg, preferably in an amount of 12.5 to 25 mg. Preferably, the stable solid pharmaceutical composition according to the present invention contains 8 mg of candesartan cilexetil and 12.5 mg of hydrochlorothiazide or 16 mg of candesartan cilexetil and 12.5 mg of hydrochlorothiazide or 32 mg of candesartan cilexetil and 12.5 mg of hydrochlorothiazide or 32 mg of candesartan cilexetil and 25 mg of hydrochlorothiazide. The term »unit dosage form« refers to physically discrete units suitable as unitary dosages for human objectes and other mammals, each unit containing a predetermined quantity of candesartan cilexetil, calculated to produce the desired therapeutic effect, in association with a suitable pharmaceutical acceptable excipient.

As used herein, the term stable refers to a solid pharmaceutical composition comprising candesartan or its pharmaceutically acceptable forms wherein the total content of impurities originating from the decomposition of candesartan does not exceed 10 % area, preferably 6 % area, more preferably 4 % area and most preferably 2 % area determined by liquid chromatography if such a composition is stored for 7 days at 60 °C in a closed vessel.

Also disclosed is a stable solid pharmaceutical composition comprising candesartan or its pharmaceutically acceptable forms wherein the total content of impurities originating from the decomposition of candesartan does not exceed 10 % area, preferably 6 % area, more preferably 4 % area and most preferably 2 % area determined by liquid chromatography if such a composition is stored for 7 days at 60 °C in a closed vessel.

Further, the term stable refers to a solid pharmaceutical composition comprising candesartan or its pharmaceutically acceptable forms wherein candesartan or its pharmaceutically acceptable forms retain their stable polymorphic form. From the state of the art it is known that the polymorphic stability of candesartan and its pharmaceutically acceptable forms in a solid pharmaceutical composition plays an important role in designing a stable pharmaceutical composition. Namely, it is known that for example candesartan cilexetil can exist in several polymorphic forms. The most stable form is known as polymorphic form I while polymorphic form II and the amorphous form are known to be not stable enough to be used in commercial products. We found that, otherwise, stable polymorphic form I of candesartan cilexetil is structurally quite weak and is prone to deformations during any physical manipulations of candesartan cilexetil in the process of preparing a pharmaceutical composition as for example during the course of mixing of the ingredients, granulating, tableting etc. If during the course of tableting some part of otherwise stable polymorphic form I of candesartan cilexetil undergoes amorphisation, this part of candesartan cilexetil will be degraded. Surprisingly, we found that the incorporation of one or more suitable plasticizer into the pharmaceutical composition remarkably lowers the friction between the individual particles of the constituents of the pharmaceutical composition during any physical manipulations of candesartan cilexetil. The consequence is that the crystal structure of polymorphic form I of candesartan cilexetil remains intact and so candesartan cilexetil retaines its polymorphic stability during and after the course of tableting.

Surprisingly, this advantage of retainment of polymorphic form I is highly expressed in case when the plasticizer is selected from the group of citrate esters. Furthermore, while citrate esters are known to be useful as plasticizers in coating procedures, we unexpectedly found that said plasticizers are useful as plasticizers in reducing friction between the constituents of the pharmaceutical composition as well.

In another aspect, the present invention relates to a process according to claim 12, for the preparation of a stable solid pharmaceutical composition comprising candesartan or its pharmaceutically acceptable forms and at least one plasticizer as described above by a process comprising a step selected from the group consisting of wet granulation, dry granulation and direct compression.

In yet another aspect of the present invention, there is provided a method of treating hypertension, cardiac diseases, cerebral apoplexy or renal diseases in a patient in need thereof by administering a stable solid pharmaceutical composition according to claims 1 to 11, comprising candesartan or its pharmaceutically acceptable forms according to the present invention.

For a more complete understanding of the instant invention, reference is made to the following illustrative examples, although it should be clearly understood that the present invention is not limited in and way thereto.

### Example 1

**Table 2. Compositions of the solid pharmaceutical composition comprising candesartan cilexetil**

| **Ingredient** | **Comparative Composition 1** | **Composition 2** | **Comparative Composition 1** | **Comparative Composition 2** |
|---|---|---|---|---|
| Candesartan cilexetil | 4.0 | 4.0 | 4.0 | 4.0 |
| Lactose monohydrate | 90.0 | 90.0 | 96.0 | 96.0 |
| Starch | 20.0 | 20.0 | 20.0 | 20.0 |
| Menthol | | 6.0 | | |
| Triethyl citrate | 6.0 | | | |
| Polyvinyl Alcohol | | | 6.0 | |
| Hydroxypropyl cellulose | 4.0 | 4.0 | 4.0 | 4.0 |
| Calcium carboxymethyl cellulose | 5.6 | 5.6 | 5.6 | 5.6 |
| Magnesium stearate | 0.4 | 0.4 | 0.4 | 0.4 |

In preparation of Comparative Composition 1, hydroxypropyl cellulose and triethyl citrate were dissolved in water and candesartan cilexetil, lactose monohydrate and starch were granulated with the aqueous hydroxypropyl cellulose / triethyl citrate solution in a fluid bed granulator. In preparation of Composition 2, hydroxypropyl cellulose was dissolved in water and candesartan cilexetil, lactose monohydrate, starch and menthol were granulated with the aqueous hydroxypropyl cellulose solution in a fluid bed granulator. In Comparative Composition 1 (WO2005/084648, example 1), hydroxypropyl cellulose and polyvinyl alcohol were dissolved in water and candesartan cilexetil, lactose monohydrate and starch were granulated with the aqueous hydroxypropyl cellulose / polyvinyl alcohol solution in a fluid bed granulator. In Comparative Composition 2 (EP 546358 B1, example 1), candesartan cilexetil, lactose monohydrate and starch were granulated in a fluid bed granulator with a dissolution of hydroxypropyl cellulose in water. After the granulation step has been performed, calcium carboxymethyl cellulose and magnesium stearate were added to the granules. One part of the obtained composition was compression-molded into tablets and the other part was filled into capsules.

**Table 3. Results of the stability test - tablets**

| **Impurities** | **Comparative Composition1** | **Comparative Composition 1** | **Comparative Composition 2** |
|---|---|---|---|
| Total content of impurities at the initial time of test (% area) | 0.4 | 0.5 | 0.8 |
| Total content after 7 days at 60 °C (% area) | 0.8 | 3.2 | 5.9 |

**Table 4. Results of the stability test - capsules**

| **Impurities** | **Comparative Composition 1** | **Comparative Composition 1** | **Comparative Composition 2** |
|---|---|---|---|
| Total content of impurities at the initial time of test (% area) | 0.4 | 0.4 | 0.5 |
| Total content after 7 days at 60 °C (% area) | 0.8 | 1.0 | 2.0 |

Table 3 shows the results of the stability test of the tablets and Table 4 shows the results for the capsules. The stability tests were performed after storing the samples for 7 days at 60 °C. The content of the candesartan cilexetil impurities was determined by liquid chromatography:
**HPLC:** Agilent 1100
**Data evaluation:** Chemstation
**Chromatographic conditions:**
Column: Eclipse Plus-C18, 50mm x 4.6mm, 1.8 µm particles
**Mobile phase:**
**Solvent A:** 0.01M NaH₂PO₄, pH 2.5
**Solvent B:** acetonitrile

**Gradient:**

| **Time (min)** | **%A** | **%B** |
|---|---|---|
| 0 | 55 | 45 |
| 12 | 5 | 95 |
| 20 | 5 | 95 |
| 21 | 55 | 45 |
| 25 | 55 | 45 |

**Post run:** 2 min
**Column temperature:** 50°C
**Flow rate:** 1.2 ml/min
**Detection:** UV, 225nm
**Injection:** 5 µl.

As it is evident from the test results, the comparative composition provides a remarkable improvement in the stability of candesartan cilexetil as compared to the other comparative compositions.

### Comparative Example 2

**Table 5. Candesartan cilexetil compositions**

| **Ingredient** | **Comparative Composition 1** | **Comparative Composition 3** | **Comparative Composition 4** | **Comparative Composition 5** | **Comparative Composition 2** |
|---|---|---|---|---|---|
| Candesartan cilexetil | 4.0 | 4.0 | 4.0 | 4.0 | 4.0 |
| Lactose monohydrate | 90.0 | 90.0 | 90.0 | 90.0 | 96.0 |
| Starch | 20.0 | 20.0 | 20.0 | 20.0 | 20.0 |
| Triethyl citrate | 6.0 | | | | |
| Gum arabic (Acacia) | | 6.0 | | | |
| Sodium Stearyl Fumarate | | | 6.0 | | |
| Sodium Lauryl Sulfate | | | | 6.0 | |
| Hydroxypropyl cellulose | 4.0 | 4.0 | 4.0 | 4.0 | 4.0 |
| Calcium carboxymethyl cellulose | 5.6 | 5.6 | 5.6 | 5.6 | 5.6 |
| Magnesium stearate | 0.4 | 0.4 | 0.4 | 0.4 | 0.4 |

In preparing Composition 1, hydroxypropyl cellulose was dissolved in water and candesartan cilexetil, lactose monohydrate, starch and menthol were granulated with the aqueous hydroxypropyl cellulose solution in a fluid bed granulator. In preparing Comparative Compositions 3, 4 and 5, gum arabic (Acacia) - as a representative of a natural gum, sodium stearyl fumarate - as a representative of a tablet or capsule lubricant, sodium lauryl sulfate - as a representative of a surfactant or an emulsifying agent were dissolved in water and candesartan cilexetil, lactose monohydrate and starch were granulated with the aqueous solution of the respective excipient in a fluid bed granulator. In preparing Comparative Composition 2 (EP 546358B1, example 1), candesartan cilexetil, lactose monohydrate and starch were granulated in a fluid bed granulator with a dissolution of hydroxypropyl cellulose in water. After the granulation step has been performed, calcium carboxymethyl cellulose and magnesium stearate were added to the granules. The obtained compositon was compression-molded into tablets.

**Table 6. Results of the stability test - tablets**

| **Impurities** | **Comparative Composition 1** | **Comparative Composition 3** | **Comparative Composition 4** | **Comparative Composition 5** | **Comparative Composition 2** |
|---|---|---|---|---|---|
| Total content of impurities at the initial time of test (% area) | 0.4 | 0.6 | 0.5 | 0.5 | 0.6 |
| Total content after 1 day at 60 °C (% area) | 0.5 | 2.1 | 1.6 | 1.1 | 1.2 |

Table 6 shows the results of the stability test of the tablets. The stability tests were performed after storing the samples for 1 day at 60 °C. The content of the candesartan cilexetil impurities was determined by liquid chromatography as mentioned above.

As it is evident from the test results, the plasticizer of the Comparative composition 1 provides a remarkable improvement in the stability of candesartan cilexetil as compared with other stabilizing agents (natural gums, lubricants and surfactants).

As it has been demonstrated in the Examples 1 and 2, a suitable plasticizer can remarkably lower the total amount of impurities originating from the decomposition of candesartan in a solid composition of candesartan cilexetil.

### Comparative Example 3

**Table 7. Composition of the stable solid pharmaceutical composition comprising candesartan cilexetil**

| **Ingredient** | **Comparative Composition 1** |
|---|---|
| Candesartan cilexetil | 32.0 |
| Lactose monohydrate | 358.0 |
| Starch | 37.0 |
| Triethyl citrate | 6.0 |
| Hydroxypropyl cellulose | 21.0 |
| Calcium carboxymethyl cellulose | 20.0 |
| Magnesium stearate | 1.5 |

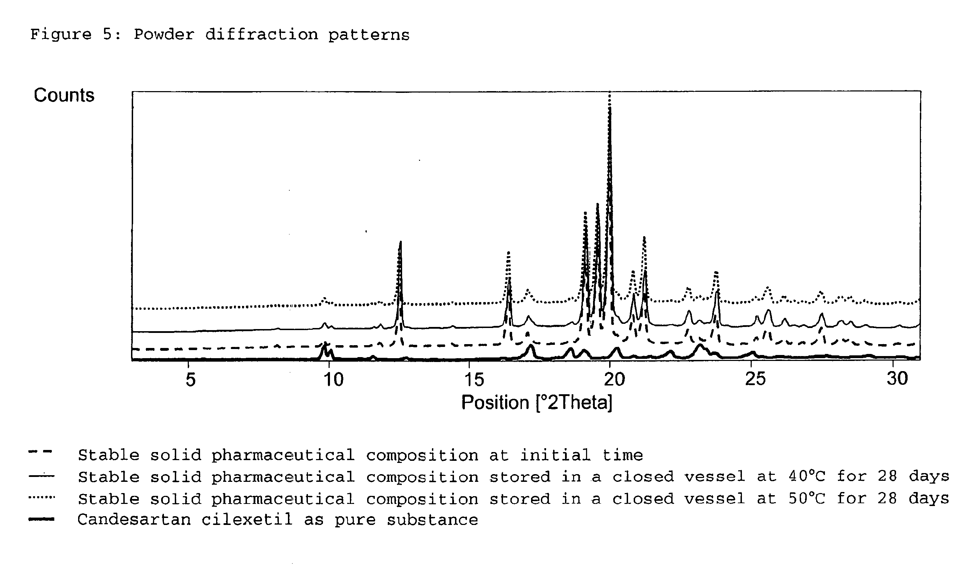

The stable solid pharmaceutical composition with ingredients as described in Table 7 was prepared by the same method as described in Example 1. The X-ray powder diffraction pattern was measured first at initial time and then after storage for 28 days at 40°C and 50°C for 28 days; the result is shown in Figure 5. The comparison between the powder diffraction patterns of stable solid pharmaceutical compositions comprising candesartan cilexetil and the powder diffraction pattern of candesartan cilexetil as pure substance, respectively shows that all characteristic peaks of polymorphic form I of candesartan cilexetil are present in the composition of the present invention. The result furthermore demonstrates that polymorphic form I of candesartan cilexetil remains unchanged as there are no additional peaks emerging after storage that would indicate the presence of any other polymorph of candesartan cilexetil.

The instrument was a PANalytical X'pert PRO powder diffractometer with CuKa radiation.

### Example 4

**Table 8. Candesartan cilexetil compositions**

| **Ingredient** | **Composition 4/1 (mg)** | **Composition 4/2 (mg)** | **Composition 4/3 (mg)** |
|---|---|---|---|
| Candesartan cilexetil | 4.0 | 4.0 | 4.0 |
| Lactose monohydrate | 90.0 | 90.0 | 90.0 |
| Starch | 20.0 | 20.0 | 20.0 |
| Dibutyl sebacate | 6.0 | 6.0 | 6.0 |
| Polysorbate 80 | / | 2.0 | |
| Sodium lauryl sulfate | / | / | 2.0 |
| Hydroxypropyl cellulose | 4.0 | 4.0 | 4.0 |
| Calcium carboxymethyl cellulose | 5.6 | 5.6 | 5.6 |
| Magnesium stearate | 0.4 | 0.4 | 0.4 |

In preparing Composition 4/1, hydroxypropyl cellulose was dissolved in water and dibutyl sebacate was dispersed into solution. Candesartan cilexetil, lactose monohydrate, starch were granulated with the obtained dispersion in a fluid bed granulator. The obtained granulate was further processed as in Example 2.

### Preparation of compositions 4/2 and 4/3:

Hydroxypropyl cellulose and polysorbate or sodium lauryl sulfate were dissolved in water and dibutyl sebacate was dispersed into solution. Candesartan cilexetil, lactose monohydrate, starch were granulated with the obtained dispersion in a fluid bed granulator. The obtained granulate was further processed as in Example 2.

### Example 5

**Table 9. candesartan cilexetil compositions**

| **Ingredient** | **Composition 5/1 (mg)** | **Composition 5/2 (mg)** | **Composition 5/3 (mg)** |
|---|---|---|---|
| Candesartan cilexetil | 4.0 | 4.0 | 4.0 |
| Lactose monohydrate | 90.0 | 90.0 | 90.0 |
| Starch | 20.0 | 20.0 | 20.0 |
| Diethyl phthalate | 6.0 | 6.0 | 6.0 |
| Polysorbate 80 | / | 2.0 | |
| Sodium lauryl sulfate | / | / | 2.0 |
| Hydroxypropyl cellulose | 4.0 | 4.0 | 4.0 |
| Calcium carboxymethyl cellulose | 5.6 | 5.6 | 5.6 |
| Magnesium stearate | 0.4 | 0.4 | 0.4 |

In preparing Composition 5/1, hydroxypropyl cellulose was dissolved in water and diethyl phthalate was dispersed into solution. candesartan cilexetil, lactose monohydrate, starch were granulated with the obtained dispersion in a fluid bed granulator. The obtained granulate was further processed as in Example 2.

### Preparation of compositions 5/2 and 5/3:

Hydroxypropyl cellulose and polysorbate or sodium lauryl sulfate were dissolved in water and diethyl phthalate was dispersed into solution. candesartan cilexetil, lactose monohydrate, starch were granulated with the obtained dispersion in a fluid bed granulator. The obtained granulate was further processed as in Example 2.

### Example 6

**Table 10. Candesartan cilexetil compositions**

| **Ingredient** | **Composition 6/1 (mg)** | **Composition 6/2 (mg)** |
|---|---|---|
| Candesartan cilexetil | 4.0 | 4.0 |
| Lactose monohydrate | 90.0 | 90.0 |
| Starch | 20.0 | 20.0 |
| Polyvinyl acetate | 6.0^{3,2} | 6.0³ |
| Hydroxypropyl cellulose | 4.0 | 4.0 |
| Calcium carboxymethyl cellulose | 5.6 | 5.6 |
| Magnesium stearate | 0.4 | 0.4 |

| | | |
|---|---|---|
| 1... Kollicoat 30SR (30% dispersion in water) 2... weight of dry substance 3... dry polymer is used | | |

In preparing of Composition 6/1, hydroxypropyl cellulose was dissolved in water. Kollicoat 30SR was admixed to the obtained solution, to obtaine a dispersion containing both polymers. Candesartan cilexetil, lactose monohydrate, starch were granulated with the obtained dispersion in a fluid bed granulator. The obtained granulate was further processed as in Example 2.

In preparing of Composition 6/2, hydroxypropyl cellulose and polyvinyl acetate were dissolved in absolute ethanole. Candesartan cilexetil, lactose monohydrate, starch were granulated with the obtained solurion in a fluid bed granulator. Obtained granulate was further processed as in Example 2.

## Claims

1. A stable solid pharmaceutical composition comprising candesartan or its pharmaceutically acceptable esters or salts and at least one plasticizer in the core, wherein the plasticizer is selected from the group consisting of phthalate esters, sebacates, polyacrylate polymers, polymers and copolymers of ethylene and/or vinyl acetate, triacetin, menthol and any mixtures thereof present in an amount of less than 20% (w/w) of the final pharmaceutical composition.

2. The stable solid pharmaceutical composition according to claim 1, wherein the plasticizer is selected from the group consisting of phthalate esters, sebacates, polyacrylate polymers, triacezin, menthol and any mixture thereof.

3. The stable solid pharmaceutical composition according to any of the preceding claims wherein the plasticizer is selected from the group consisting of dibutyl sebacates, diethyl phthalate polyvinyl acetate, menthol and triacetin and any mixture thereof.

4. The stable solid pharmaceutical composition according to any of the preceding claims further comprising at least one pharmaceutically acceptable ingredient selected from the group consisting of one or more diluent, one or more binder, one or more disintegrant, one or more surfactant, one or more lubricant.

5. The stable solid pharmaceutical composition according to any of the preceding claims wherein one or more diluent is selected from the group consisting of microcrystalline cellulose, powdered cellulose, lactose, sugar, sugar alcohols, siliconised microcrystalline cellulose, calcium hydrogenphosphate, calcium carbonate, calcium lactate or any mixtures thereof.

6. The stable solid pharmaceutical composition according to any of the preceding claims wherein one or more binder is selected from the group consisting of polyvinylpyrrolidone, microcrystalline cellulose, hydroxyethylcellulose, hydroxypropylcellulose, hydroxypropylmethylcellulose or other cellulose ethers, starch, pregelatinised starch, or polymethacrylate, or any mixtures thereof.

7. The stable solid pharmaceutical composition according to any of the preceding claims wherein one or more disintegrant is selected from the group consisting of crospovidone, starch, sodium starch glycollate, microcrystalline cellulose, carboxymethylcellulose sodium or calcium, cross-linked carboxymethylcellulose sodium, polacrilin potassium, low-substituted hydroxypropylcellulose or any mixtures thereof.

8. The stable solid pharmaceutical composition according to any of the preceding claims wherein one or more surfactant is selected from the group consisting of anionic or cation surfactant, ampholytic surfactants and nonionic surfactants or any mixtures thereof.

9. The stable solid, pharmaceutical composition according to any of the preceding claims wherein one or more lubricant is selected from the group consisting of stearic acid or stearic acid salts, such as for example magnesium stearate, magnesium palmitate, magnesium oleate, hydrogenated vegetable oil, hydrogenated castor oil, talc, sodium stearyl fumarate, macrogols or any mixtures thereof.

10. The stable solid pharmaceutical composition according to any of the preceding claims further comprising a film coating.

11. The stable solid pharmaceutical composition according to any of the preceding claims wherein the stable solid pharmaceutical composition is in the form of tablets, pills, powders, lozenges, sachets, soft and hard gelatine capsules or suppositories, preferably in the form of tablets or capsules.

12. A process for preparing a stable solid pharmaceutical composition according to any preceding claims comprising a step selected from the group consisting of wet granulation, dry granulation and direct compression.

13. Use of a plasticizer selected from the group consisting of sebacates as a stabilizing agent in the core of a solid pharmaceutical composition containing candesartan or a pharmaceutically acceptable salt or ester thereof, particularly candesartan cilexetil, as an active ingredient.

14. The use according to claim 13, wherein the plasticizer is dibutyl sebacate.

15. Stable solid pharmaceutical composition according to claims 1 to 11 for use in a method for treating hypertension, cardiac diseases, cerebral apoplexy or renal diseases.

## Patentansprüche

1. Stabile feste pharmazeutische Zusammensetzung, umfassend Candesartan oder dessen pharmazeutisch annehmbare(n/s) Ester oder Salz und mindestens einen Weichmacher im Kern, wobei der Weichmacher aus der Gruppe bestehend aus Phthalatestern, Sebacaten, Polyacrylatpolymeren, Polymeren und Copolymeren von Ethylen und/oder Vinylacetat, Triacetin, Menthol und jeder Mischung davon ausgewählt ist und in einer Menge von weniger als 20 % (G/G) der endgültigen pharmazeutischen Zusammensetzung vorhanden ist.

2. Stabile feste pharmazeutische Zusammensetzung gemäss Anspruch 1, wobei der Weichmacher aus der Gruppe bestehend aus Phthalatestern, Sebacaten, Polyacrylatpolymeren, Triacetin, Menthol und jeder Mischung davon ausgewählt ist.

3. Stabile feste pharmazeutische Zusammensetzung gemäss einem der vorstehenden Ansprüche, wobei der Weichmacher aus der Gruppe bestehend aus Dibutylsebacaten, Diethylphthalat, Polyvinylacetat, Menthol, Triacetin und jeder Mischung davon ausgewählt ist.

4. Stabile feste pharmazeutische Zusammensetzung gemäss einem der vorstehenden Ansprüche, die ferner mindestens einen pharmazeutisch annehmbaren Bestandteil, ausgewählt aus der Gruppe bestehend aus einem oder mehreren Verdünnungsmittel(n), einem oder mehreren Bindemittel(n), einem oder mehreren Sprengmittel(n), einem oder mehreren Tensid(en) und einem oder mehreren Gleitmittel(n), umfasst.

5. Stabile feste pharmazeutische Zusammensetzung gemäss einem der vorstehenden Ansprüche, wobei das eine oder die mehreren Verdünnungsmittel aus der Gruppe bestehend aus mikrokristalliner Cellulose, pulverförmiger Cellulose, Lactose, Zucker, Zuckeralkoholen, siliconisierter mikrokristalliner Cellulose, Calciumhydrogenphosphat, Calciumcarbonat, Calciumlactat oder jeder Mischung davon ausgewählt ist/sind.

6. Stabile feste pharmazeutische Zusammensetzung gemäss einem der vorstehenden Ansprüche, wobei das eine oder die mehreren Bindemittel aus der Gruppe bestehend aus Polyvinylpyrrolidon, mikrokristalliner Cellulose, Hydroxyethylcellulose, Hydroxypropylcellulose, Hydroxypropylmethylcellulose oder anderen Celluloseethern, Stärke, vorgelatinisierter Stärke oder Polymethacrylat oder jeder Mischung davon ausgewählt ist/sind.

7. Stabile feste pharmazeutische Zusammensetzung gemäss einem der vorstehenden Ansprüche, wobei das eine oder die mehreren Sprengmittel aus der Gruppe bestehend aus Crospovidon, Stärke, Natriumstärkeglykolat, mikrokristalliner Cellulose, Natrium- oder Calciumcarboxymethylcellulose, vernetzter Natriumcarboxymethylcellulose, Polacrilinkalium, niedrigsubstituierter Hydroxypropylcellulose oder jeder Mischung davon ausgewählt ist/sind.

8. Stabile feste pharmazeutische Zusammensetzung gemäss einem der vorstehenden Ansprüche, wobei das eine oder die mehreren Tensid(e) aus der Gruppe bestehend aus einem anionischen oder kationischen Tensid, ampholytischen Tensiden und nicht-ionischen Tensiden oder jeder Mischung davon ausgewählt ist/sind.

9. Stabile feste pharmazeutische Zusammensetzung gemäss einem der vorstehenden Ansprüche, wobei das eine oder die mehreren Gleitmittel aus der Gruppe bestehend aus Stearinsäure oder Stearinsäuresalzen, wie beispielsweise Magnesiumstearat, Magnesiumpalmitat, Magnesiumoleat, hydriertem Pflanzenöl, hydriertem Rizinusöl, Talk, Natriumstearylfumarat, Macrogolen oder jeder Mischung davon ausgewählt ist/sind.

10. Stabile feste pharmazeutische Zusammensetzung gemäss einem der vorstehenden Ansprüche, die ferner eine Filmbeschichtung aufweist.

11. Stabile feste pharmazeutische Zusammensetzung gemäss einem der vorstehenden Ansprüche, wobei die stabile feste pharmazeutische Zusammensetzung in Form von Tabletten, Pillen, Pulvern, Lutschtabletten, Sachets, Weich- und Hartgelatinekapseln oder Suppositorien, vorzugsweise in Form von Tabletten oder Kapseln, vorliegt.

12. Verfahren zur Herstellung einer stabilen festen pharmazeutischen Zusammensetzung gemäss einem der vorstehenden Ansprüche, das einen Schritt, ausgewählt aus der Gruppe bestehend aus Nassgranulieren, Trockengranulieren und Direktverpressen, umfasst.

13. Verwendung eines Weichmachers, ausgewählt aus der Gruppe bestehend aus Sebacaten, als Stabilisierungsmittel im Kern einer festen pharmazeutischen Zusammensetzung, enthaltend Candesartan oder ein(en) pharmazeutisch annehmbare(s/n) Salz oder Ester davon, insbesondere Candesartan cilexetil, als Wirkstoff.

14. Verwendung gemäss Anspruch 13, wobei der Weichmacher Dibutylsebacat ist.

15. Stabile feste pharmazeutische Zusammensetzung gemäss Ansprüchen 1 bis 11 zur Verwendung in einem Verfahren zur Behandlung von Bluthochdruck, Herzkrankheiten, Gehirnschlag oder Nierenerkrankungen.

## Revendications

1. Composition pharmaceutique solide stable comprenant du candésartan ou ses esters ou sels pharmaceutiquement acceptables et au moins un plastifiant dans le noyau, où le plastifiant est choisi dans le groupe constitué des esters de phtalate, des sébacates, des polymères de polyacrylate, des polymères et des copolymères d'éthylène et/ou d'acétate de vinyle, de la triacétine, du menthol et de mélanges quelconques de ceux-ci, présent en une quantité inférieure à 20% (en poids) de la composition pharmaceutique finale.

2. Composition pharmaceutique solide stable selon la revendication 1, dans laquelle le plastifiant est choisi dans le groupe constitué des esters de phtalate, des sébacates, des polymères de polyacrylate, de la triacétine, du menthol et d'un mélange quelconque de ceux-ci.

3. Composition pharmaceutique solide stable selon l'une des revendications précédentes, dans laquelle le plastifiant est choisi dans le groupe constitué des sébacates de dibutyle, du phtalate de diéthyle, de l'acétate de polyvinyle, du menthol et de la triacétine et d'un mélange quelconque de ceux-ci.

4. Composition pharmaceutique solide stable selon l'une des revendications précédentes, comprenant en outre au moins un ingrédient pharmaceutiquement acceptable choisi dans le groupe constitué d'un ou de plusieurs diluant(s), d'un ou de plusieurs liant(s), d'un ou de plusieurs délitant(s), d'un ou de plusieurs tensioactif(s), d'un ou de plusieurs lubrifiant(s).

5. Composition pharmaceutique solide stable selon l'une des revendications précédentes, dans laquelle un ou plusieurs diluant(s) est/sont choisi(s) dans le groupe constitué de la cellulose microcristalline, de la cellulose en poudre, du lactose, du sucre, des alcools glucidiques, de la cellulose microcristalline siliconée, de l'hydrogénophosphate de calcium, du carbonate de calcium, du lactate de calcium ou de mélanges quelconques de ceux-ci.

6. Composition pharmaceutique solide stable selon l'une des revendications précédentes, dans laquelle un ou plusieurs liant(s) est/sont choisi(s) dans le groupe constitué de la polyvinylpyrrolidone, de la cellulose microcristalline, de l'hydroxyéthylcellulose, de l'hydroxypropylcellulose, de l'hydroxypropylméthylcellulose ou d'autres éthers de cellulose, de l'amidon, de l'amidon prégélatinisé, ou du polyméthacrylate, ou de mélanges quelconques de ceux-ci.

7. Composition pharmaceutique solide stable selon l'une des revendications précédentes, dans laquelle un ou plusieurs délitant(s) est/sont choisi(s) dans le groupe constitué de la crospovidone, de l'amidon, du glycolate d'amidon sodique, de la cellulose microcristalline, de la carboxyméthylcellulose sodique ou calcique, de la carboxyméthylcellulose sodique réticulée, de la polacriline de potassium, de l'hydroxypropylcellulose faiblement substituée ou de mélanges quelconques de ceux-ci.

8. Composition pharmaceutique solide stable selon l'une des revendications précédentes, dans laquelle un ou plusieurs tensioactif(s) est/sont choisi(s) dans le groupe constitué d'un tensioactif anionique ou cationique, de tensioactifs ampholytes et de tensioactifs non ioniques ou de mélanges quelconques de ceux-ci.

9. Composition pharmaceutique solide stable selon l'une des revendications précédentes, dans laquelle un ou plusieurs lubrifiant(s) est/sont choisi(s) dans le groupe constitué de l'acide stéarique ou des sels de l'acide stéarique, tels que, par exemple, le stéarate de magnésium, le palmitate de magnésium, l'oléate de magnésium, l'huile végétale hydrogénée, l'huile de ricin hydrogénée, le talc, le stéarylfumarate de sodium, les macrogols ou des mélanges quelconques de ceux-ci.

10. Composition pharmaceutique solide stable selon l'une des revendications précédentes, comprenant en outre un revêtement pelliculaire.

11. Composition pharmaceutique solide stable selon l'une des revendications précédentes, dans laquelle la composition pharmaceutique solide stable se présente sous la forme de comprimés, de pilules, de poudres, de pastilles, de sachets, de capsules de gélatine molles et dures ou de suppositoires, de préférence sous la forme de comprimés ou de capsules.

12. Processus de préparation d'une composition pharmaceutique solide stable selon l'une des revendications précédentes, comprenant une étape choisie dans le groupe constitué par la granulation par voie humide, la granulation par voie sèche et la compression directe.

13. Utilisation d'un plastifiant choisi dans le groupe constitué par les sébacates en tant qu'agent stabilisant dans le noyau d'une composition pharmaceutique solide contenant du candésartan ou l'un de ses sels ou esters pharmaceutiquement acceptables, en particulier du candésartan cilexétil, en tant que principe actif.

14. Utilisation selon la revendication 13, dans laquelle le plastifiant est le sébacate de dibutyle.

15. Composition pharmaceutique solide stable selon les revendications 1 à 11, destinée à être utilisée dans un procédé pour le traitement de l'hypertension, des maladies cardiaques, de l'apoplexie cérébrale ou des maladies rénales.
